# EUROPÄISCHE PATENTSCHRIFT

(11) **EP 3 648 813 B1**
(45) Veröffentlichungstag und Bekanntmachung des Hinweises auf die Patenterteilung: **26.07.2023**
(21) Anmeldenummer: 18735286.9
(22) Anmeldetag: 28.06.2018
(51) Int. Cl.: A61M 1/28, A61M 39/10

(54) **VORRICHTUNG ZUR ERKENNUNG DES KONNEKTIONSZUSTANDES**
DEVICE FOR DETECTING THE STATE OF CONNECTION
DISPOSITIF DE DETECTION DE L'ETAT DE CONNEXION

(30) Priorität: 03.07.2017 DE 102017114800
(43) Veröffentlichungstag der Anmeldung: 13.05.2020
(73) Patentinhaber: Fresenius Medical Care Deutschland GmbH, 61352 Bad Homburg (DE)
(72) Erfinder: HOCHREIN, Torsten, 97478 Eschenau (Knetzgau) (DE); SAAL, Stefan, 97453 Schonungen (DE); HEDMANN, Frank, 97332 Volkach (DE)
(74) Vertreter: Herrmann, Uwe
(86) Internationale Anmeldenummer: PCT/EP2018/067489
(87) Internationale Veröffentlichungsnummer: WO 2019/007816

(56) Entgegenhaltungen:
- EP-A1- 1 494 737
- WO-A1-2008/106452
- WO-A1-2015/162603
- US-A1- 2009 007 642
- US-A1- 2014 262 252
- US-B1- 6 851 427

## Beschreibung

Die vorliegende Erfindung betrifft eine Vorrichtung zur Erkennung des Konnektionszustandes zwischen einem Patientenkatheter und einem Schlauch-Kassettensystem, das zur Verwendung in einem Dialysegerät bestimmt ist.

Bei der Peritonealdialyse wird der im Patient implantierte Patientenkatheter mit einem Disposable verbunden, durch das die Dialyselösung zum Patientenkatheter und durch diesen in den Bauchraum des Patienten gelangt. Das Disposable dient ferner dazu, die Dialyselösung nach einer bestimmten Verweildauer aus dem Bauraum abzuleiten und in einen Drainagebeutel zu leiten.

Bei dem Disposable handelt es sich um einen Einwegartikel, der nach der Behandlung verworfen wird.

Ein Problem bei bestehenden Peritonealdialysebehandlungen besteht darin, dass der Konnektionszustand zwischen dem Patientenkatheter und dem Disposable nicht zuverlässig erkannt werden kann. Bekannte Peritonealdialysegeräte können ein Lösen der mechanischen Verbindung, die üblicherweise als Drehverbindung ausgebildet ist, des Patientenkonnektors nicht erkennen. Dies kann zur Folge haben, dass das Gerät unter Umständen durch die offene Stelle im Disposable kein Dialysat, sondern verkeimte Fremdluft ansaugt.

Bei bekannten Peritonealdialyseanwendungen muss der Patient dem Peritonealdialysegerät, in Folgenden auch einfach als "Gerät" bezeichnet, mitteilen, ob er über das Schlauchsystem, d.h. über das Disposable mit dem Gerät verbunden ist. Diese Vorgehensweise ist vergleichsweise umständlich und fehleranfällig, da sie eine Interaktion zwischen dem Patienten und dem Gerät erfordert. Im Übrigen ist die Überprüfung der korrekten Konnektion mit dem Gerät durch den Patienten auch insofern problematisch, als dass durch die Berührung des Gerätes bzw. des Disposables die Gefahr einer Verkeimung besteht. Bei einer Nachjustage des Disposables können somit ggf. Keime in das Schlauchsystem eindringen, was unbedingt zu vermeiden ist, da diese Keime ggf. zu einer Entzündung des Peritoneums führen können oder andere unerwünschte Nebenwirkungen haben können.

US 2009/007642 A1 offenbart ein Dialysat-Prüfverfahren, umfassend die Anordnung elektrischer Kontakte in einem Dialysat-Flussweg, die Messung einer elektrischen Eigenschaft des Dialysats und die Bestimmung, ob das Dialysat anhand der festgestellten elektrischen Eigenschaft richtig gemischt wurde. WO 2008/106452 A1 betrifft eine Kassette zur Durchführung der Peritonealdialyse umfassend ein Sensorapparatesystem zur Bestimmung einer oder mehrerer Eigenschaften der Dialysatlösung in einer Kassette.

Der vorliegenden Erfindung liegt daher die Aufgabe zugrunde, eine Vorrichtung zur Ermittlung des Konnektionszustandes des Patienten mit dem Disposable bereitzustellen, die keine Aktion des Patienten erfordert und die automatisiert erfolgen kann.

Diese Aufgabe wird durch eine Vorrichtung mit den Merkmalen des Anspruchs 1 gelöst. Danach ist vorgesehen, dass die Vorrichtung das Schlauch-Kassettensystem und einen elektrischen Schaltkreis aufweist, der an dem Schlauch-Kassettensystem sowie an dem Patientenkatheter angeordnet ist und dass die Vorrichtung des Weiteren eine Messvorrichtung zur Messung wenigstens einer elektrischen Eigenschaft des elektrischen Schaltkreises aufweist, die von dem genannten Konnektionszustand abhängt.

Der vorliegenden Erfindung liegt somit der Gedanke zugrunde, das Disposable sowie einen Abschnitt des Patientenkatheters, im Folgenden auch "Katheterabschnitt" genannt, mit einem elektrischen Leiter bzw. mit einem Schaltkreis zu versehen, mittels dessen geprüft werden kann, ob die Konnektion des Patienten mit dem Gerät bzw. mit dem Disposable ordnungsgemäß erfolgt ist oder nicht. Der Begriff "Patientenkatheter" umfasst auch eine etwaige Katheterverlängerung.

Die Begriffe "Schlauchsystem", "Disposable" und "Schlauch-Kassettensystem" werden im Rahmen der vorliegenden Erfindung synonym verwendet und kennzeichnen den Leitungsabschnitt zwischen dem implantierten Patientenkatheter und dem Peritonealdialysegerät. Dieser Leitungsabschnitt umfasst üblicherweise eine im Gerät angeordnete Kassette, von der sich die Patientenleitung erstreckt. Diese ist üblicherweise über einen Patientenkonnektor mit dem Patientenkatheter verbunden. Die vorliegende Erfindung ist aber nicht auf eine derartige Anordnung beschränkt, sondern umfasst jedes beliebige Disposable, mittels dessen frische Dialyselösung zum Patienten gefördert werden kann und/oder verbrauchte Dialyselösung vom Patienten abgezogen werden kann.

Durch die Messvorrichtung, die vorzugsweise im Peritonealdialysegerät angeordnet ist, kann wenigstens ein elektrischer Parameter des genannten Schaltkreises gemessen werden, wobei über die Größe des Parameters Rückschlüsse auf den Konnektionszustand gezogen werden können.

Vorzugsweise handelt es sich bei dem elektrischen Parameter um den elektrischen Widerstand. In Betracht kommen alternativ oder zusätzlich jedoch auch andere elektrische Größen, wie z.B. die Stromstärke, die Spannung, die Impedanz etc.

Der Patientenkatheter umfasst ein Dioden-Widerstandsnetzwerk.

Des Weiteren umfasst das Schlauch-Kassettensystem einen Patientenkonnektor, der mit einem Dioden-Widerstandsnetzwerk und einem parallel angeordneten Schalter versehen ist.

Im behandlungsfertigen Zustand ist der Patientenkonnektor mit dem Konnetkor des Patientenkatheters verbunden.

Die Diode des Dioden-Widerstandsnetzwerkes des Katheterabschnitts kann antiparallel zu der Diode des Dioden-Widerstandsnetzwerkes des Patientenkonnektors angeordnet sein.

In einer weiteren Ausgestaltung der Erfindung kann das Schlauch-Kassettensystem eine Patientenleitung umfassen, die mit einer Kontaktleitung versehen ist.

Vorzugsweise umfasst das Schlauch-Kassettensystem einen Kassettenabschnitt, der mit Kontaktstellen auf der Oberfläche des Kassettenabschnitts versehen ist. Beispielsweise kann durch Verpressen der Kassette mit den im Maschinenblock des Gerätes eingearbeiteten Kontakten eine elektrische Verbindung zwischen dem Gerät und der Kassette und damit mit dem Disposable hergestellt werden. Dazu kann die Kassettenfolie bzw. die Kassettenoberfläche beispielsweise mit einer leitenden Farbe oder sonstigen leitenden Beschichtung bedruckt oder beschichtet werden.

Gemäß der Erfindung ist vorgesehen, dass die Messvorrichtung ausgebildet ist, den Widerstand des Dioden-Widerstandsnetzwerkes des Katheterabschnittes zu messen und auf die Diskonnektion des Patienten bzw. des Patientenkatheters zu schließen, wenn dieser Widerstand unendlich ist. So kann auf vergleichsweise einfache Art und Weise ermittelt werden, ob der Patient noch mit dem Gerät verbunden ist oder nicht.

Denkbar ist es weiterhin, den Widerstand des Dioden-Widerstandsnetzwerkes des Patientenkonnektors zu messen und auf einen geschlossenen Patientenkonnektor zu schließen, wenn aufgrund des dann geschlossenen Schalters ein Kurzschluss gemessen wird. Somit kann durch eine einfache Messung ermittelt werden, ob der Patientenkonnektor verschlossen ist oder nicht. Ist der Patientenkonnektor verschlossen, blockiert dieser die Leitung zwischen der Kassette und dem Patienten, so dass kein Durchfluss durch das Disposable möglich ist.

Die Messvorrichtung kann ausgebildet sein, das Fehlen eines Widerstands des Dioden-Widerstandsnetzwerks eines Patientenkonnektors zu erfassen und so auf das Entfernen des Patientenkonnektors zu schließen. So kann überprüft werden, ob und - im Falle mehrerer in Reihe angeordneter Patientenkonnektoren - welcher Patientenkonnektor für eine Diskonnektion genutzt wurde.

Bei einem Patientenkonnektor handelt es sich vorzugsweise um einen Konnektor, wie er in der DE 198 14 047 C1 beschrieben ist. Auf den Offenbarungsgehalt dieser Druckschrift wird insoweit Bezug genommen. Vorzugsweise ist vorgesehen, dass der Schalter des Dioden-Widerstandsnetzwerks geschlossen wird, wenn der Konnektor geschlossen wird.

In einer weiteren Ausgestaltung der Erfindung ist vorgesehen, dass die Messvorrichtung ausgebildet ist, durch die Erfassung einer elektrischen Eigenschaft des Schlauch-Kassettensystems auf die Art des Schlauch-Kassettensystems zu schließen. Um eine eindeutige Entscheidung z.B. zwischen einem Kassetten-Schlauchset für die Standardbehandlung und einer pädiatrischen Behandlung sicherstellen zu können, ist es somit auch denkbar, dass durch eine Widerstandskodierung der Kassette oder einer Anordnung elektrisch leitender Elemente an verschiedenen Positionen eine Unterscheidung zwischen verschiedenen Arten von Disposables sicher vorgenommen werden kann.

Vorzugsweise ist die Messvorrichtung ausgebildet, die Messung automatisiert durchzuführen. Eine Eingreifen des Patienten oder eine Interaktion mit dem Patienten ist in diesem Falle nicht erforderlich.

Es kann eine Anzeigevorrichtung vorgesehen sein, die mit der Messvorrichtung verbunden ist und die ausgebildet ist, das von der Messvorrichtung ermittelte Ergebnis der Messung oder eine darauf basierende Information auszugeben. So kann beispielsweise aus der Widerstandsmessung die Meldung generiert werden "Patient nicht mit Gerät verbunden", "Patientenkonnektor nicht konnektiert", Patientenkonnektor verschlossen" etc.

Die vorliegende Erfindung betrifft des Weiteren ein Dialysegerät, insbesondere ein Peritonealdialysegerät, wobei das Dialysegerät mit einer Vorrichtung gemäß einem der Ansprüche 1 bis 11 ausgerüstet ist.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente umfassen können. Ebenso schließt die Verwendung des Plurals auch das Vorhandensein des fraglichen Elementes in der Einzahl ein und umgekehrt umfasst der Singular auch mehrere der fraglichen Elemente.

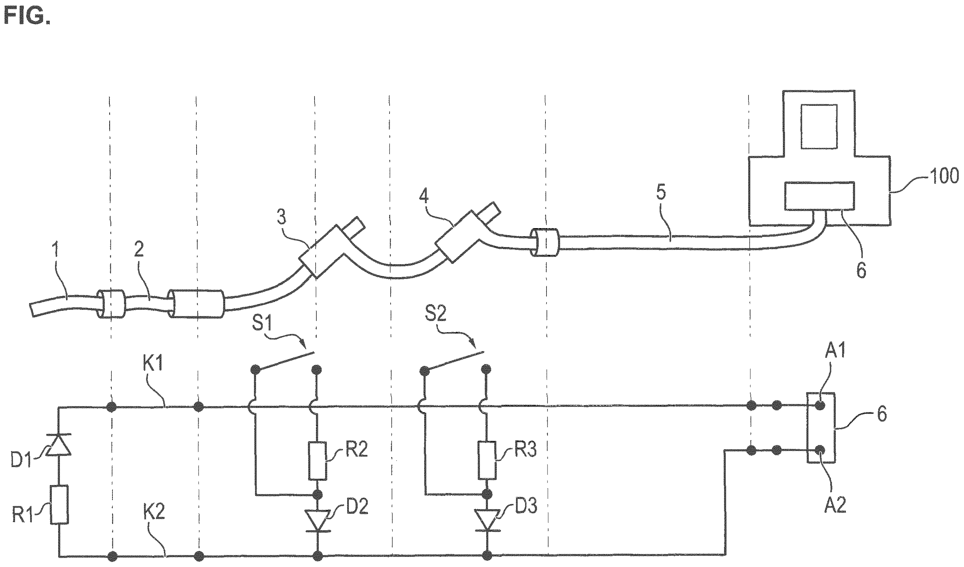

Weitere Einzelheiten und Vorteile der Erfindung werden anhand eines in der Zeichnung dargestellten Ausführungsbeispiels näher erläutert:
Die einzige Figur zeigt eine Ansicht eines Disposables sowie eines Abschnitts des Patientenkatheters im oberen Bereich der Figur und darunter den elektrischen Schaltkreis, mit dem das Dispoable sowie der Patientenkatheter versehen ist.

Das Disposable mit Patientenkatheter ist in sechs Abschnitte wie folgt unterteilt:
Bezugszeichen 1: Patientenkatheter,
Bezugszeichen 2: Patientenkatheterverlängerung,
Bezugszeichen 3: erster Patientenkonnektor,
Bezugszeichen 4: zweiter Patientenkonnektor,
Bezugszeichen 5: Patientenleitung,
Bezugszeichen 6: Set bzw. Kassette im Gerät

Wie dies weiter aus der Figur hervorgeht, ist der Katheter 1, d.h. der Patientenkatheter, der mit einem Abschnitt in den Patienten implantiert ist, mit einer Reihenschaltung aus Widerstand R1 und Diode D1 versehen, die zwischen zwei parallel angeordneten Kontaktleitungen K1 und K2 verlaufen. Diese Kontaktleitungen stehen an den Anschlussstellen A1 und A2 mit der Kassette 6 in Verbindung. Die Anschlussstellen A1 und A2 stehen in leitendem Kontakt mit zugehörigen Kontakten des Gerätes 100, so dass die Leitungen K1 und K2 durch das Gerät 100 mit Strom bzw. Spannung beaufschlagt werden können.

Das Disposable, d.h. der Einmalartikel umfasst die Abschnitte 3 bis 6.

Die Katheterverlängerung 2 erstreckt sich zwischen dem Patientenkatheter und ist mit einer Durchführung der Kontaktleitungen K1 und K2 versehen.

Die Patientenkonnektoren 3 und 4 sind jeweils mit einem Dioden-Widerstandsnetzwerk und einem Schalter S1 und S2 versehen, wie dies aus der Figur hervorgeht. Die jeweiligen Widerstände R2 und R3 sind mit den jeweiligen Dioden D2 und D3 in Reihe geschaltet und verlaufen zwischen den Kontaktleitungen K1 und K2, wobei die Dioden D2 und D3 antiparallel zu der Diode D1 verschaltet sind. Der Patientenkonnektor 3 steht mit dem Patientenkatheter 1 bzw. dessen Verlängerung 2 in Kontakt, so dass Dialyselösung von dem Disposable zu dem Patientenkatheter oder umgekehrt von dem Patientenkatheter zu dem Disposable strömen kann.

Die Patientenleitung 5 ist mit einer Durchführung der Kontaktleitungen K1 und K2 versehen.

Die Kassette 6 weist wie oben ausgeführt Anschlussstellen A1 und A2 für die Kontaktleitungen K1 und K2 auf.

Durch diese Anordnung sind durch eine nicht näher dargestellte Messvorrichtung folgende Zustände erfassbar:

### I. Beim Start des Gerätes

a) Widerstand R1 wird ausgemessen, Patient ist noch mit dem Gerät verbunden,
b) Widerstand R2 II R3 wird ausgemessen, beide Patientenkonnektoren sind unverschlossen,
c) Widerstand R3 wird ausgemessen, Patientenkonnektor 4 ist unverschlossen,
d) Kurzschlussmessung, zumindest ein Patientenkonnektor ist verschlossen.

### II. Beim Aufrüsten des Gerätes

b) Widerstand R2 II R3 wird ausgemessen, beide Patientenkonnektoren sind unverschlossen,
c) Widerstand R3 wird ausgemessen, Patientenkonnektor 4 ist unverschlossen. Aus dieser Information kann die Füllmenge in der Patientenleitung 5 bestimmt werden. Dies kann durch eine kapazitive Messung erreicht werden.

Denkbar ist es beispielsweise, dass Luft als Referenz herangezogen wird und basierend darauf durch eine kapazitive Messung erfasst werden kann, wie groß die Füllmenge an Dialyselösung in der Patientenleitung ist.

Aufgrund der Tatsache, dass jede Lösung eine bestimmte Dielektrizitätskonstante aufweist, kann durch die Messung der Dielektrizitätskonstante zudem ermittelt werden, ob dem Patienten die richtige, d.h. vorgeschriebene Peritonealdialyselösung zugeführt wird. Somit kann festgestellt werden, ob der Patient mit der korrekten Lösung behandelt wird und dies ggf. dem Nutzer bzw. Patienten angezeigt werden. Ebenso kann eine Warnmeldung abgegeben und/oder die Behandlung unterbrochen oder nicht gestartet werden, wenn sich die nicht die vorgeschriebene Lösung in der Patientenleitung befindet.

### III. Während der Behandlung

a) Widerstand R1 wird ausgemessen, Patient ist noch mit dem Gerät verbunden,
b) Widerstand R1 zeigt unendlich hohen Widerstand, Patient ist nicht mehr mit dem Gerät verbunden,

### IV. Erkennung des Entfernens des zweiten Patientenkonnektors 4

a) Widerstand R3 wird ausgemessen, R2 wurde entfernt.

### V. Diskonnektion

### Schritt 1:

a) Kurzschlussmessung, ein Patientenkonnektor ist verschlossen,
b) Widerstand R1 wird ausgemessen, Patient ist noch mit dem Gerät verbunden.

### Schritt 2:

a) Kurzschlussmessung, ein Patientenkonnektor ist verschlossen,
b) Widerstand R1 zeigt unendlich hohen Widerstand, Patient ist nicht mehr mit dem Gerät verbunden.

Die Messvorrichtung ist ausgebildet, die o.g. Messungen vollautomatisch durchzuführen und das Ergebnis der Messungen an eine Anzeigeeinrichtung zu übermitteln. Auf dieser wird sodann für den Nutzer optisch und/oder akustisch wahrnehmbar das Ergebnis der Messung oder eine darauf basierende Information ausgegeben.

## Patentansprüche

1. Vorrichtung zur Erkennung des Konnektionszustandes zwischen einem Patientenkatheter (1) und einem Schlauch-Kassettensystem, das zur Verwendung in einem Dialysegerät (100) bestimmt ist, **dadurch gekennzeichnet, dass** die Vorrichtung das Schlauch-Kassettensystem und einen elektrischen Schaltkreis aufweist, der an dem Schlauch-Kassettensystem sowie an dem Patientenkatheter (1) angeordnet ist und dass die Vorrichtung des Weiteren eine Messvorrichtung zur Messung wenigstens einer elektrischen Eigenschaft des elektrischen Schaltkreises aufweist, die von dem genannten Konnektionszustand abhängt, dass der Schaltkreis ein Dioden-Widerstandsnetzwerk aufweist, das an dem Patientenkathether (1) angeordnet ist, dass das Schlauch-Kassettensystem einen Patientenkonnektor (3, 4) umfasst, der mit einem Dioden-Widerstandsnetzwerk und einem parallel angeordneten Schalter (S1, S2) versehen ist, und dass die Messvorrichtung ausgebildet ist, den Widerstand des Dioden-Widerstandsnetzwerkes des Patientenkatheters (1) und den Widerstand des Dioden-Widerstandsnetzwerkes des Patientenkonnektors (3, 4) zu messen.

2. Vorrichtung nach Anspruch 1, **dadurch gekennzeichnet, dass** es sich bei der elektrischen Eigenschaft um den elektrischen Widerstand handelt.

3. Vorrichtung nach Anspruch 1, **dadurch gekennzeichnet, dass** die Diode (D1) des Dioden-Widerstandsnetzwerkes des Patientenkatheters (1) antiparallel zu der Diode (D2, D3) des Dioden-Widerstandsnetzwerkes des Patientenkonnektors (3, 4) angeordnet ist.

4. Vorrichtung nach einem der vorhergehenden Ansprüche, **dadurch gekennzeichnet, dass** das Schlauch-Kassettensystem eine Patientenleitung umfasst, die mit einer Kontaktleitung (K1, K2) versehen ist.

5. Vorrichtung nach einem der vorhergehenden Ansprüche, **dadurch gekennzeichnet, dass** das Schlauch-Kassettensystem einen Kassettenabschnitt umfasst, der mit Kontaktstellen auf der Oberfläche des Kassettenabschnitts versehen ist.

6. Vorrichtung nach einem der vorhergehenden Ansprüche, **dadurch gekennzeichnet, dass** die Messvorrichtung ausgebildet ist, den Widerstand des Dioden-Widerstandsnetzwerkes des Patientenkatheters (1) zu messen und auf die Diskonnektion des Patienten zu schließen, wenn dieser Widerstand unendlich ist.

7. Vorrichtung nach einem der vorhergehenden Ansprüche, **dadurch gekennzeichnet, dass** die Messvorrichtung ausgebildet ist, den Widerstand des Dioden-Widerstandsnetzwerkes des Patientenkonnektors (3, 4) zu messen und auf einen geschlossenen Patientenkonnektor (3, 4) zu schließen, wenn ein Kurzschluss gemessen wird.

8. Vorrichtung nach einem der vorhergehenden Ansprüche, **dadurch gekennzeichnet, dass** die Messvorrichtung ausgebildet ist, das Fehlen eines Widerstands des Dioden-Widerstandsnetzwerks eines Patientenkonnektors (3, 4) zu messen und so auf das Entfernen des Patientenkonnektors zu schließen.

9. Vorrichtung nach einem der vorhergehenden Ansprüche, **dadurch gekennzeichnet, dass** die Messvorrichtung ausgebildet ist, durch die Erfassung einer elektrischen Eigenschaft des Schlauch-Kassettensystems auf die Art des Schlauch-Kassettensystems zu schließen.

10. Vorrichtung nach einem der vorhergehenden Ansprüche, **dadurch gekennzeichnet, dass** die Messvorrichtung ausgebildet ist, die Messung automatisiert durchzuführen.

11. Vorrichtung nach einem der vorhergehenden Ansprüche, **dadurch gekennzeichnet, dass** eine Anzeigevorrichtung vorgesehen ist, die mit der Messvorrichtung verbunden ist und die ausgebildet ist, das von der Messvorrichtung ermittelte Ergebnis der Messung oder eine darauf basierende Information auszugeben.

12. Dialysegerät, insbesondere Peritonealdialysegerät, **dadurch gekennzeichnet, dass** das Dialysegerät mit einer Vorrichtung gemäß einem der Ansprüche 1 bis 11 ausgerüstet ist.

## Claims

1. Device for detecting the state of connection between a patient catheter (1) and a tubing cassette system, which is designed for use in a dialysis machine (100), **characterised in that** the device comprises the tubing cassette system and an electrical circuit that is arranged on the tubing cassette system and on the patient catheter (1), and **in that** the device further comprises a measuring device for measuring at least one electrical property of the electrical circuit, which depends on the state of connection, **in that** the circuit comprises a diode resistive network arranged on the patient catheter (1), **in that** the tubing cassette system comprises a patient connector (3, 4) provided with a diode resistive network and a switch (S1, S2) arranged in parallel, and **in that** the measuring device is designed to measure the resistivity of the diode resistive network of the patient catheter (1) and the resistivity of the diode resistive network of the patient connector (3, 4).

2. Device according to claim 1, **characterised in that** the electrical property is the electrical resistivity.

3. Device according to claim 1, **characterised in that** the diode (D1) of the diode resistive network of the patient catheter (1) is arranged anti-parallel to the diode (D2, D3) of the diode resistive network of the patient connector (3, 4).

4. Device according to any one of the preceding claims, **characterised in that** the tubing cassette system comprises a patient line, which is provided with a contact line (K1, K2).

5. Device according to any one of the preceding claims, **characterised in that** the tubing cassette system comprises a cassette portion, which is provided with contact points on the surface of the cassette portion.

6. Device according to any one of the preceding claims, **characterised in that** the measuring device is designed to measure the resistivity of the diode resistive network of the patient catheter (1) and to derive disconnection of the patient if said resistivity is infinite.

7. Device according to any one of the preceding claims, **characterised in that** the measuring device is designed to measure the resistivity of the resistive network of the patient connector (3, 4) and to derive a locked patient connector (3, 4) if a short circuit is measured.

8. Device according to any one of the preceding claims **characterised in that** the measuring device is designed to measure lack of resistivity of the diode resistivity network of a patient connector (3, 4) and to derive removal of the patient connector.

9. Device according to any one of the preceding claims, **characterised in that** the measuring device is designed to derive the type of the tubing cassette system by the detection of an electrical property of the tubing cassette system.

10. Device according to any one of the preceding claims, **characterised in that** the measuring device is designed to perform measurement in an automated manner.

11. Device according to any one of the preceding claims, **characterised in that** a display device is provided, to which the measuring device is connected and which is designed to output the result of the measurement or any information based thereon, determined by the measuring device.

12. Dialysis machine, in particular a peritoneal dialysis machine, **characterised in that** the dialysis machine is provided with a device according to any one of claims 1 to 11.

## Revendications

1. Dispositif de détection de l'état de connexion entre un cathéter de patient (1) et un système de cassette tubulaire destiné à être utilisé dans un appareil de dialyse (100), **caractérisé en ce que** le dispositif présente le système de cassette tubulaire et un circuit électrique qui est agencé sur le système de cassette tubulaire ainsi que sur le cathéter de patient (1) et **en ce que** le dispositif présente en outre un dispositif de mesure pour mesurer au moins une propriété électrique du circuit électrique, qui dépend dudit état de connexion, **en ce que** le circuit présente un réseau de résistance à diodes agencé sur le cathéter de patient (1), **en ce que** le système de cassette tubulaire comprend un connecteur de patient (3, 4), qui est muni d'un réseau de résistance à diodes et d'un commutateur (S1, S2) agencé en parallèle, et **en ce que** le dispositif de mesure est configuré pour mesurer la résistance du réseau de résistance à diodes du cathéter de patient (1) et la résistance du réseau de résistance à diodes du connecteur de patient (3, 4).

2. Dispositif selon la revendication 1, **caractérisé en ce que** la propriété électrique consiste en la résistance électrique.

3. Dispositif selon la revendication 1, **caractérisé en ce que** la diode (D1) du réseau de résistance à diodes du cathéter de patient (1) est agencée de manière antiparallèle à la diode (D2, D3) du réseau de résistance à diodes du connecteur de patient (3, 4).

4. Dispositif selon l'une quelconque des revendications précédentes, **caractérisé en ce que** le système de cassette tubulaire comprend une ligne de patient qui est munie d'une ligne de contact (K1, K2).

5. Dispositif selon l'une quelconque des revendications précédentes, **caractérisé en ce que** le système de cassette tubulaire comprend une section de cassette munie de points de contact sur la surface de la section de cassette.

6. Dispositif selon l'une quelconque des revendications précédentes, **caractérisé en ce que** le dispositif de mesure est configuré pour mesurer la résistance du réseau de résistance à diodes du cathéter de patient (1) et pour conclure à la déconnexion du patient lorsque cette résistance est infinie.

7. Dispositif selon l'une quelconque des revendications précédentes, **caractérisé en ce que** le dispositif de mesure est configuré pour mesurer la résistance du réseau de résistance à diodes du connecteur de patient (3, 4) et pour conclure à un connecteur de patient fermé (3, 4) lorsqu'un court-circuit est mesuré.

8. Dispositif selon l'une quelconque des revendications précédentes, **caractérisé en ce que** le dispositif de mesure est configuré pour mesurer l'absence d'une résistance du réseau de résistance à diodes d'un connecteur de patient (3, 4) et pour conclure ainsi à l'enlèvement du connecteur de patient.

9. Dispositif selon l'une quelconque des revendications précédentes, **caractérisé en ce que** le dispositif de mesure est configuré pour déduire le type de système de cassette tubulaire en détectant une propriété électrique du système de cassette tubulaire.

10. Dispositif selon l'une quelconque des revendications précédentes, **caractérisé en ce que** le dispositif de mesure est configuré pour effectuer la mesure de manière automatique.

11. Dispositif selon l'une quelconque des revendications précédentes, **caractérisé en ce qu'**il est prévu un dispositif d'affichage qui est relié au dispositif de mesure et qui est configuré pour émettre le résultat de la mesure déterminé par le dispositif de mesure ou une information basée sur celui-ci.

12. Appareil de dialyse, notamment appareil de dialyse péritonéale, **caractérisé en ce que** l'appareil de dialyse est équipé d'un dispositif selon l'une quelconque des revendications 1 à 11.
